# EUROPEAN PATENT APPLICATION

(11) **EP 3 936 111 A1**
(43) Date of publication of application: **12.01.2022**
(21) Application number: 21397508.9
(22) Date of filing: 02.07.2021
(51) Int. Cl.: A61K 8/9789, A23L 2/08, A61Q 1/00, A61Q 5/00, A61Q 19/00, B01D 5/00, C02F 1/02

(54) **USE OF BERRY FRACTIONS IN COSMETIC COMPOSITIONS**

(30) Priority: 03.07.2020 FI 20205714
(71) Applicant: Lumene Oy, 02780 Espoo (FI)
(72) Inventor: ISOHANNI, Tiina, 02780 Espoo (FI); MÄKINEN, Päivi, 02780 Espoo (FI); PESONEN, Terhi, 02780 Espoo (FI); PALMUJOKI, Ingela, 02780 Espoo (FI); VÄNTTINEN, Kristofer, 02780 Espoo (FI); SAHRAMO, Elina, 02780 Espoo (FI)
(74) Representative: Laine IP Oy

(57) **Abstract**

According to an example aspect of the present invention, there is provided a cosmetic composition comprising cosmetically acceptable substances and at least one cosmetically acceptable berry fraction, wherein the at least one cosmetically acceptable berry fraction comprises a berry industry side stream or an extract thereof.

## Description

### FIELD

The present invention relates to the use of berry fractions, obtained as side streams of berry industry, in cosmetic compositions. In particular, the invention relates to the use of berry waters, obtained as side streams or by-products in the production of berry juice and berry juice related products, in cosmetic compositions. The invention also relates to compositions and methods for treating and improving the appearance of skin, wherein the compositions include berry fractions obtained as side streams of berry industry. The berry fractions are preferably derived from Nordic berries.

### BACKGROUND

Production processes in food industry, including berry industry, are more automated and enable larger production volumes as before. When production volumes grow, more by-products are created. There is no exact, universal definition for a "by-product". However, many publications define by-products as raw materials or parts of raw materials, which are not included in the final food products. The utilization of side streams, waste streams or by-products is primarily guided by costs.

Berry industry traditionally produces juices, jams, marmalades and alcoholic products, but the number of new products such as seed oils, dried berry products and frozen purees has increased during recent years.

Berries have had an important role in human diet for ages and nowadays also their health effects have been widely recognized. The health effects are often connected to vitamins, fiber and the phenolic compounds found in abundance in berries. Many phenolic compounds are well known antioxidants but they also protect berries against hazards from their environment, e.g. from excess UV radiation of the sun or against attacks of viruses, bacteria and fungi. Also the colors of berries are influenced by the combinations of different phenolic compounds in each berry species. These compounds are thus mostly located on the skin layers of the berries.

Phenolic compounds found in berries include for example hydroxybenzoic acids, hydroxycinnamic acids, anthocyanins, flavonols, flavan-3-ols, proanthocyanidins, gallic acid, ellagic acid and hydrolysable tannins. Quantitatively, anthocyanins and ellagitannins are the predominant phenolic compound classes in berries. Among hydroxycinnamic acids found in berries, p-coumaric acid is the major one. Quercetin on the other hand is the major flavonol in several berries.

Berries are also rich in vitamins, in particularly vitamin C and vitamin E. Among wild berries, sea buckthorn and cloudberry are especially good sources of both vitamin C and vitamin E. Vitamin A in berries occurs in its precursor forms, carotenoids.

Berry juice production is a common practice to utilize berries in an industrial scale. The juice is pressed from berries and the skins and seeds are left in the press residue, which is also called a press cake. The press cake is highly perishable material as such, but drying extends its usefulness and shelf life. A big challenge for the berry industry has been to find profitable use for this side stream, which may form up to 30% of the original weight of the berries. The seeds are to some extent used to make berry seed oils, and the fibrous berry skins as a berry fiber powder are used in food applications. Still, plenty of press residue and hence significant amounts of berry phenolic compounds remain unutilized.

Berry juice pressing typically produces approximately 80-90% raw berry juice of the original weight of the berries, depending on the particular berry and its moisture %. However, the yield of this raw berry juice may vary from 70 to 90% of the original weight of the berries. After pressing, the raw berry juice is concentrated, thus removing for example half of the water from the raw berry juice, typically by evaporation, to obtain concentrated berry juice. The concentrated berry juice is used in the production processes of the berry industry. The vaporized water, that contains small amounts of berry substance, is condensated. The condensated vaporized water is typically discharged as a waste stream.

One option for further processing of the concentrated berry juice is spray-drying. Recently, spray-dried berry juice has found increasing use in various applications, including cosmetic compositions. Spray-dried berry powders are typically prepared by using maltodextrin as carrying material. Dehydration of berry juice may thus be carried out in a suitable spray dryer, by feeding mixtures of concentrated berry juice and maltodextrin into the spray dryer, wherein hot air is employed as drying vehicle. The resulting dry berry powder is water-soluble and finds use in various health foods, dietary supplements, as well as in cosmetic products.

In a spray dryer the liquid input stream (a mixture of concentrated berry juice and carrier) is sprayed through a nozzle into a hot vapor stream and vaporized. The solid part (the dried powder) is collected, typically in a cyclone or a drum. The solvent that is vaporized into the drying gas is taken away and optionally circulated. However, as part of the berry substances is vaporized in the drying gas, the drying gas partly tends to condensate on the walls of the drying chamber and flows to the bottom of the drying chamber as a condensated vaporized berry water, wherefrom it is typically discharged.

A common trend nowadays is towards using natural ingredients in cosmetic products. Therefore, the cosmetic industry is constantly searching for new safe ingredients of plant origin. In addition, these ingredients should be produced cost effectively with minimal and simple processing and should maintain their activity in final products. At the same time companies aim at sustainable development and sustainable use of natural resources.

In patent literature various patent applications disclose methods for extracting, isolating and enriching berry fractions from berries. Also various berry waters are increasingly popular. For instance, WO 2018/055244 A1 discloses a makeup-fixing cosmetic composition comprising 1-30 wt-% blueberry water, together with some other plant based extracts, such as rose water. US 5,853,730 relates to cosmetic cleansing solutions wherein citrus water distillate provides a citrus odor to the cosmetic.

However, it has now been found that certain berry industry side streams are useful as such or after only minor modification steps, such as concentration, different types of extraction processes, pressing, fermentation, and filtration, in cosmetic compositions. Alternatively, said side streams may be used as sources of suitable active compounds such as phenolic compounds, which may be isolated and/or enriched to be used in specific applications. The present invention shows an option to make use of side streams or waste streams from berry industry, particularly from concentration of berry juices, for cosmetic applications.

### SUMMARY OF THE INVENTION

The invention is defined by the features of the independent claims. Some specific embodiments are defined in the dependent claims.

The present invention is based on the concept of using side streams or by-products from berry industry in cosmetic compositions. In particular, the invention takes advantage of concentration residues from berry juice processing, in particular aqueous evaporation residues (condensated vaporized waters) from berry juice processing,.

According to a first aspect of the present invention, there is thus provided a cosmetic composition comprising cosmetically acceptable substances and at least one cosmetically acceptable berry fraction, wherein the at least one cosmetically acceptable berry fraction comprises or is a berry industry side stream from processing of Nordic berries. Typically said side stream is a an evaporation residue from concentration of berry juice or an evaporation residue from spray drying of berry juice, in particular condensated vaporized water from concentration of berry juice and/or condensated vaporized water from spray drying of berry juice.

According to a second aspect of the present invention, there is provided a method for utilizing side streams of berry industry wherein said side streams are residues from concentration of berry juice, selected from condensated vaporized water from a concentration process of raw berry juice to concentrated berry juice, condensated vaporized water from spray drying of concentrated berry juice, and their combinations, and wherein said side streams are included in cosmetic compositions as such or after modification steps selected from concentration, different types of extraction processes, fermentation, filtration and/or adding preservatives.

Embodiments of the invention comprise cosmetic compositions wherein the side stream, waste product or by-product is selected from an evaporation residue from a concentration process of raw berry juice to concentrated berry juice or an evaporation residue from spray drying of concentrated berry juice.

Considerable advantages are obtained by the invention. First, the invention provides use to otherwise discarded or only partly utilized by-product materials of berry industry. The berries involved may include all berries grown in Nordic nature and used in production processes of berry industry.

Second, the invention provides cosmetic composition wherein the amount of cosmetically acceptable berry fractions may be high, in some products such as skin oils or tonic cosmetic waters even 99.5% (w/w) of the total weight of the cosmetic compositions. In certain products, such as skin oils or tonic cosmetic waters, berry waters obtained as residues from berry juice concentration, may replace water otherwise included in the product formula.

Third, as disclosed in the present application, the side streams or by-products from berry industry can be included in various types of cosmetic compositions, including but not limited to skin care creams, skin care lotions, skin care gels, skin oils, cosmetic tonic waters, cleansing gels, foundation creams, lip products, zinc oxide based creams, roll-on antiperspirants, mascaras, shampoos, conditioners, hair masks, hair sprays, hair waxes, hair gels, hair mousses, hair pomaces, hair coloring compositions (permanent, semi-permanent, or direct dye), permanent lotions, oxidizing agents for coloring of hair, heat protection compositions for hair, or beard and moustache cleansing, conditioning or styling products.

Further features and advantages of the present technology will appear from the following description of some embodiments.

### EMBODIMENTS

### DEFINITIONS

In the present context, the term "side stream" or "by-product" or "waste stream" refers to berry raw materials or parts of berry raw materials which are not included in the final products of berry industry. In general, the terms may comprise at least the following berry industry side streams or by-products or extracts thereof: berry waters, berry extracts, berry oils, berry vinegars, berry vinegar extracts, dry berry extracts, and berry crush.

Within this disclosure, "berry water" refers to an aqueous concentration residue from concentration process of berry juice or to an aqueous evaporation residue from spray drying of berry juice. When raw berry juice, obtained from pressing of berries, is concentrated, usually approximately half of the water in the raw berry juice is removed, typically by evaporation. The removed water fraction contains small amounts of berry substances and is called "berry water". Further, within this disclosure "berry water" refers also to an aqueous evaporation residue from spray drying of concentrated berry juice.

Within this disclosure, "berry extracts" refer to water-soluble or oil-soluble extracts from berry pressing waste. Said berry extracts may be dried and are then referred as "dry berry extracts".

Within this context, "berry oils" includes oil-soluble extracts from berry pressing waste.

Within this disclosure, "berry vinegars" and "berry vinegar extracts" refer to fermented extracts of berry leaves or to fermented extracts of press residue from berry juice production.

Within this context, "berry crush" refers to dried and crushed peel, skin or seed wastes from berry industry.

"Berries" include but are not limited to wild Nordic berries, *i.e.* berries naturally growing in the Nordic area. The Nordic area refers to a geographical region in Northern Europe and the North Atlantic. Preferably, the "Nordic berries" referred herein include berries growing in the Nordic countries, which by definition include Denmark, Finland, Iceland, Norway, and Sweden, as well as Greenland, the Faroe Islands, the Åland islands and Svalbard archipelagos.

The berries are preferably wild harvested. However, to ensure diversity of Nordic nature, sources of natural berry extracts and oils may also be cultured. The natural ingredients may thus originate from organic farming methods or from wild harvesting in compliance with national legislation.

Nordic berries thus include but are not limited to lingonberry (*Vaccinium vitis-idaea*), bilberry (*Vaccinium myrtillus*), cloudberry (*Rubus chamaemorus*), cranberry (*Vaccinium oxycoccus* / *Oxycoccus palustris*), small cranberry *(Vaccinium microcarpum),* bog bilberry (*Vaccinium uliginosum*)*,* raspberry (*Rubus idaeus*), crowberry (*Empetrum nigrum*), rowanberry (*Sorbus aucuparia, Sorbus fennica*), sea buckthorn (*Hippophaë rhamnoides*), blackcurrant (*Ribes nigrum*), redcurrant (*Ribes rubrum*)*,* white currant (*Ribes rubrum* f. *leucocarpum*), greencurrant (*Ribes nigrum*)*,* gooseberry (*Ribes uva-crispa*), wild red currant (*Ribes spicatum*), mountain currant (*Ribes alpinum*)*,* aronia berry (*Aronia mitschurinii*)*,* saskatoon (*Amelanchier alnifolia*)*,* Arctic bramble (*Rubus arcticus*)*,* stone bramble (*Rubus saxatilis*)*,* strawberry (*Fragaria x ananassa*)*,* blackberry (*Rubus fruticosus*)*,* bearberry (*Arctostaphylos uva-ursi*), alpine bearberry (*Arctostaphylos alpine*)*,* wild strawberry (*Fragaria vesca*), juniper berry (*Juniperus communis*)*,* wild rose (*Rosa acicularis*)*,* dog rose (*Rosa canina*), cinnamon rose (*Rosa cinnamomea* / *Rosa majalis*), glaucous dog rose (*Rosa dumalis*)*,* blackthorn (*Prunus spinosa*)*,* Cornelian cherry (*Cornus mas*), bunchberry (*Cornus suecica*)*,* dewberry (*Rubus caesius*), Finnish whitebeam (*Hedlundia hybrida*) and bird cherry (*Prunus padus*)*.*

As stated above, in the present invention side streams or by-products from berry industry, in particular berry waters, are included in cosmetic compositions. In particular, the side streams or by-products may be included in the cosmetic compositions as such or essentially as such, without laborious multi-step chemical extraction and isolation processes that would involve extensive use of chemicals, typically hazardous solvents. Typically, berry industry side streams, in particular berry waters, are included in the cosmetic compositions of the invention as such or after subjecting them to one or more of the following process steps: filtration, concentration, different types of extraction processes, fermentation, adding solvents, typically solvents such as alcohol, natural oils, and adding preservatives.

In one embodiment, the process steps are selected from the list of chemical and biological processes that may be used to manufacture derived natural, derived organic and derived mineral ingredients according to ISO standard no. 16128-1, Annex B.

The cosmetic composition according to the invention comprises cosmetically acceptable substances and at least one cosmetically acceptable berry fraction, wherein said at least one cosmetically acceptable berry fraction comprises at least one berry industry side stream from processing of Nordic berries.

In a preferred embodiment the cosmetic composition according to the invention comprises cosmetically acceptable substances and at least one cosmetically acceptable berry fraction, wherein said at least one cosmetically acceptable berry fraction consists of or consists essentially of at least one berry industry side stream from processing of Nordic berries.

In particular, the berry industry side stream or by-product is a residue from concentration of berry juice, preferably an aqueous residue from concentration of berry juice.

The berry industry side streams or extracts therefrom thus comprise berry waters, obtained as side streams of berry industry as indicated above.

In one embodiment the berry industry side stream is a residue from concentration of berry juice, preferably an evaporation residue from concentration of berry juice or an evaporation residue from spray drying of berry juice. Said side streams are also referred to as berry waters.

In one embodiment, the berry industry side stream is an evaporation residue from concentration of berry juice, typically from the concentration of raw berry juice. Thus in one embodiment the berry industry side stream is berry water obtained as an aqueous evaporation residue from concentration of berry juice.

In one preferred embodiment, the berry industry side stream is berry water obtained as an aqueous evaporation residue from spray drying of berry juice, in particular from spray drying a mixture of berry juice and maltodextrin.

The berry industry side streams used in the present invention may also be combined with other berry industry side streams or extracts therefrom. Typical examples include i) berry water and berry crush, ii) berry water, berry crush and berry oil, and iii) berry water, berry crush, berry oil and berry vinegar extract.

The berries may be selected from among the berries disclosed above as Nordic berries. In one embodiment of the invention, the berries are selected from the group consisting of lingonberry (*Vaccinium vitis-idaea*), bilberry (*Vaccinium myrtillus*), cloudberry (*Rubus chamaemorus*)*,* cranberry (*Vaccinium oxycoccus* / *Oxycoccus palustris*)*,* small cranberry (*Vaccinium microcarpum*), crowberry (*Empetrum nigrum*), and sea buckthorn (*Hippophaë rhamnoides*), preferably from lingonberry and cloudberry.

According to the invention the side streams or by-products from berry industry may be included in several types of cosmetic compositions. Typically, the cosmetic composition according to the invention is selected from the group consisting of a skin care cream, skin care lotion, skin care gel, skin oil, cosmetic tonic water, cleansing gel, a foundation cream, a lip care stick, a lip care cream, a lip care gel, a lip colour stick, cream or gel, zinc oxide based cream, a roll-on antiperspirant, a mascara, shampoo, conditioner, hair mask, hair spray, hair wax, hair gel, hair mousse, hair pomace, hair coloring composition (permanent, semi-permanent, or direct dye), permanent lotion, oxidizing agent for coloring of hair, heat protection composition for hair, or a beard and moustache cleansing, conditioning or styling product. In some embodiments, skin care creams, skin care lotions, skin care gels, skin oils, cosmetic tonic waters, cleansing gels, foundation creams, zinc oxide based creams, roll-on antiperspirants, mascaras are preferred.

The cosmetic composition according to the invention may contain an amount of cosmetically acceptable berry fraction(s) consisting of or consisting essentially of berry industry side streams, which is at most 99.5 % (w/w) of the total weight of the cosmetic composition.

In further embodiments, the cosmetic composition according to the invention may contain an amount of cosmetically acceptable berry fraction(s), which consist of or consist essentially of berry industry side streams or extracts therefrom, in particular berry waters, wherein the amount is at least 25%, preferably at least 50%, more preferably at least 75%, of the total weight of the cosmetic composition.

In an embodiment the cosmetic composition is a skin care cream, oil, lotion or gel, a cosmetic tonic water, a cleansing gel, a make-up product, or an antiperspirant roll-on, wherein the amount of cosmetically acceptable berry fraction(s) as defined herein is 0.01-99.5% (w/w), preferably 0.01-95%, 0.01-70%, 0.01-60%, 0.01-50%, 0.01-40%, 0.01-30%, 0.01-20%, 0.01-10% or 0.01-5%, of the total weight of the composition.

In one embodiment, the cosmetic composition is a zinc oxide based cream, wherein the amount of cosmetically acceptable berry fraction(s) is 0.01-60% (w/w), preferably 0.01-50%, 0.01-40%, 0.01-30%, 0.01-20%, 0.01-10%, 0.01-5% or 0.1-5%, of the total weight of the composition.

In an embodiment, the cosmetic composition is a skin oil, wherein the amount of cosmetically acceptable berry fraction(s) is 0.01-99.5% (w/w), preferably 0.01-80%, 0.01-70%, 0.01-60%, 0.01-50%, 0.01-40%, 0.01-30% or 0.5-20%, of the total weight of the composition.

In a further embodiment the cosmetic composition is a lip care or lip colour product, such as a stick, cream or gel, wherein the amount of cosmetically acceptable berry fraction(s) is 0.01-20% (w/w), preferably 0.01-15%, 0.01-10%, 0.01-5%, 0.01-3% or 0.01-2%, of the total weight of the composition.

In another embodiment the cosmetic composition is a foundation cream, wherein the amount of cosmetically acceptable berry fraction(s) is 0.01-90% (w/w), preferably 0.01-80%, 0.01-70%, 0.01-60%, 0.01-50%, 0.01-40%, 0.01-30%, 0.01-20%, 0.01-10% or 0.01-5%, of the total weight of the composition.

In one embodiment the cosmetic composition is a mascara, wherein the amount of cosmetically acceptable berry fraction(s) is 0.01-80% (w/w), preferably 0.01-50%, 0.01-40%, 0.01-30%, 0.01-20%, 0.01-10%, 0.01-5% or 0.1-5%, of the total weight of the composition.

In some embodiments the cosmetic composition may be a hair care product, such as shampoo, conditioner, hair mask, hair spray, hair wax, hair gel, hair mousse, hair pomace, hair coloring composition (permanent, semi-permanent, or direct dye), permanent lotion, oxidizing agent for coloring of hair, or heat protection composition for hair, or a beard and moustache cleansing, conditioning or styling product, wherein the amount of cosmetically acceptable berry fraction(s) is 0.01-80% (w/w), preferably 0.01-50%, 0.01-40%, 0.01-30%, 0.01-20%, 0.01-10%, 0.01-5% or 0.1-5%, of the total weight of the composition.

Cosmetically acceptable substances that may be included in the cosmetic compositions include but are not limited to cosmetically acceptable humectants, thickeners, bulking agents, UV filters, emulsifying agents, surfactants, emollients, preservatives, antimicrobial agents, colorants, film forming agents, flavouring agents, antioxidants, foam boosting agents, parfum, oils, waxes and fats, alcohols, and water.

It is to be understood that the embodiments of the invention disclosed are not limited to the particular structures, process steps, or materials disclosed herein, but are extended to equivalents thereof as would be recognized by those ordinarily skilled in the relevant arts. It should also be understood that terminology employed herein is used for the purpose of describing particular embodiments only and is not intended to be limiting.

Reference throughout this specification to one embodiment or an embodiment means that a particular feature, structure, or characteristic described in connection with the embodiment is included in at least one embodiment of the present invention. Thus, appearances of the phrases "in one embodiment" or "in an embodiment" in various places throughout this specification are not necessarily all referring to the same embodiment. Where reference is made to a numerical value using a term such as, for example, about or substantially, the exact numerical value is also disclosed.

As used herein, a plurality of items, structural elements, compositional elements, and/or materials may be presented in a common list for convenience. However, these lists should be construed as though each member of the list is individually identified as a separate and unique member. Thus, no individual member of such list should be construed as a de facto equivalent of any other member of the same list solely based on their presentation in a common group without indications to the contrary. In addition, various embodiments and example of the present invention may be referred to herein along with alternatives for the various components thereof. It is understood that such embodiments, examples, and alternatives are not to be construed as de facto equivalents of one another, but are to be considered as separate and autonomous representations of the present invention.

Furthermore, the described features, structures, or characteristics may be combined in any suitable manner in one or more embodiments. In the following description, numerous specific details are provided, such as examples of lengths, widths, shapes, etc., to provide a thorough understanding of embodiments of the invention. One skilled in the relevant art will recognize, however, that the invention can be practiced without one or more of the specific details, or with other methods, components, materials, etc. In other instances, well-known structures, materials, or operations are not shown or described in detail to avoid obscuring aspects of the invention.

### EXPERIMENTAL

### Example 1. Skin care cream

An example of ingredients for a skin care cream, lotion or gel composition for facial and throat skin care is shown in the following Table 1. The described skin care cream, lotion or gel composition moisturizes, brightens and softens the skin. In addition, the disclosed composition may possess antioxidant properties due to the high amount of berry based ingredients from side streams of berry industry.

**Table 1. Skin care cream, skin care lotion, skin care gel**

| INGREDIENTS | Max. levels % (w/w) |
|---|---|
| Oils, waxes and fats | 30 |
| Humectants | 20 |
| Thickeners | 12 |
| Ethanol (alcohol denat.) | 10 |
| Berry based ingredients | 0.01-99.5 |
| Bulking agents | 5 |
| UV filters | 5 |
| Emulsifying agents | 5 |
| Preservatives, antimicrobials | 2 |
| Colorants | 2 |
| Perfume | 1 |
| Aqua | to 100 |

In the above composition and in the following examples preferred ingredients include for example the following unless otherwise indicated: Preferred oils include various vegetable oils, such as sweet almond oil, canola oil, avocado oil, naturally-derived plant esters. Preferred fats include for example long chain alcohols and plant waxes. Humectants include e.g. glycerine and propylene glycol. Examples of thickeners are for example carbomer and xanthan gum. Berry based ingredients include berry waters, berry extracts, berry vinegar extracts, berry oils, dry berry extracts, berry crush, which are side streams of berry industry as defined above, and any combinations thereof, in particular berry waters. Berry waters may also replace water otherwise included in the composition. Emulsifying agents include anionic, amphoteric and non-ionic surfactants, such as PEG stearate, ceteareth, cetearyl glucoside, sucrose stearate/distearate, sodium stearoyl glutamate, sorbitan olivate, glyceryl stearate citrate.

### Example 2. Zinc oxide based cream with UV filter

| INGREDIENTS | Max. levels % (w/w) |
|---|---|
| Oils, waxes and fats | 60 |
| Bulking agents | 30 |
| Zinc oxide | 35 |
| Humectants | 20 |
| Emulsifying agents | 12 |
| Berry based ingredients | 0.01-60 |
| Thickeners | 5 |
| Ethanol (alcohol denat.) | 5 |
| Preservatives, antimicrobials | 1.5 |
| Perfume | 1 |
| Aqua | to 100 |

In the above composition bulking agents may include e.g. titaniumdioxide and silica.

### Example 3. Skin oil

| INGREDIENTS | Max. levels % (w/w) |
|---|---|
| Ethanol (alcohol denat.) | 90 |
| Humectants (e.g. glycerin) | 20 |
| Berry based ingredients | 99.5-100 |
| Thickeners (e.g. dextrin palmitate) | 5 |
| Perfume | 5 |
| Additional ingredients | 5 |
| Preservatives, antimicrobials | 1.5 |
| Colorants | 1 |
| Oils (e.g. vegetable) | to 100 |

In the above composition, "additional ingredients" include for example antioxidants and aqua.

### Example 4. Tonic cosmetic water

| INGREDIENTS | Max. levels % (w/w) |
|---|---|
| Ethanol (alcohol denat.) | 70 |
| Humectants | 25 |
| Berry based ingredients | 95 |
| Oils (e.g. vegetable oils) | 50 |
| Additional ingredients | 5 |
| Emulsifying agents | 5 |
| Thickeners | 2 |
| Preservatives, antimicrobials | 2 |
| Perfume | 1.5 |
| Colorants | 1 |
| Aqua | to 100 |

In the above composition, humectants include for example glycerin and propylene glycol. "Additional ingredients" include for example vitamins and UV filters. Emulsifying agents include anionic, amphoteric or non-ionic surfactants, for example sorbitan stearate. Thickeners include for example carbomer and xanthan gum.

### Example 5. Lip care product or lip colour product (stick, cream or gel)

| INGREDIENTS | Max. levels % (w/w) |
|---|---|
| Oils, waxes and fats | 90 |
| Humectants | 50 |
| Aqua | 50 |
| Film forming agents | 30 |
| Thickeners | 15 |
| UV filters | 15 |
| Bulking agents (e.g. silica) | 10 |
| Emulsifying agents | 5 |
| Cosmetic colorants | 5 |
| Ethanol (alcohol denat.) | 5 |
| Berry based ingredients | 0.01-20 |
| Perfume | 20 |
| Preservatives, antimicrobials | 1 |
| Flavouring agents | 1 |

In the above composition, humectants include for example glycerin and propylene glycol. An example of film forming agents is VP/hexadecane copolymer. Waxes include e.g. carnauba and candelilla cera. Typical thickeners in the above composition include for example carbomer and hydroxyethylcellulose. Examples of emulsifying agents include for example trioleyl phosphate and glyceryl dibehenate.

### Example 6. Cleansing gel

| INGREDIENTS | Max. levels % (w/w) |
|---|---|
| Anionic surfactants | 20 |
| Oils, waxes and fats | 20 |
| Humectants | 20 |
| Non-ionic / amphoteric surfactants | 15 |
| Foam boosting agents | 5 |
| Berry based ingredients | 0.01-95 |
| Thickeners | 5 |
| Preservatives, antimicrobials | 2 |
| Perfume | 1 |
| Colorants | 1 |
| Aqua | to 100 |

In the above composition, examples of anionic surfactants are sodium lauroyl sarcosinate and sodium laureth sulfate. Humectants include for example glycerine and propylene glycol. Non-ionic or amphoteric surfactants include for example betaine derivatives and glucose derivatives. An example of foam boosting agents is cocamide MEA (monoethanolamine). An example of suitable thickeners for the above composition is hydroxypropyl methylcellulose.

### Example 7. Antiperspirant roll-on

| INGREDIENTS | Max. levels % (w/w) |
|---|---|
| Ethanol (alcohol denat.) | 65 |
| Oils and waxes | 30 |
| Antiperspirant salts | 25 |
| Thickeners, emollients, emulsifying agents | 25 |
| Bulking agents | 10 |
| Berry based ingredients | 0.01-95 |
| Deodorants | 5 |
| Perfume | 3 |
| Preservatives, antimicrobials | 1.5 |
| Aqua | to 100 |

In the above composition, typical examples of oils and waxes are vegetable oils and vegetable esters. Antiperspirant salts include for example aluminium salts and aluminium/zirconium complexes. Examples of bulking agents include perlite and silica. A typical deodorant is for example zinc ricinoleate.

### Example 8. Foundation emulsion

| INGREDIENTS | Max. levels % (w/w) |
|---|---|
| Liquid paraffin and isoparaffin | 60 |
| Oils | 40 |
| Emollients (e.g. plant esters) | 30 |
| Silicones | 30 |
| Bulking agents | 30 |
| Colorants, colour additives | 30 |
| Humectants | 25 |
| Thickeners, emulsifying agents | 20 |
| Ethanol (alcohol denat.) | 15 |
| UV filters | 10 |
| Surfactants | 5 |
| Berry based ingredients | 0.01-90 |
| Perfume | 1 |
| Preservatives, antimicrobials | 1 |
| Aqua | to 100 |

In the above composition, suitable isoparaffins include for example branched-chain isoparaffin (C11-C16), isododecane and isohexadecane. Typical bulking agents include for example talc, kaolin, silica and starch. Colour additives may include for example opacifying agents and pearlescent agents. Surfactants may be anionic, amphoteric and/or non-ionic surfactants.

### Example 9. Mascara

| INGREDIENTS | Max. levels % (w/w |
|---|---|
| Liquid paraffin and isoparaffin | 80 |
| Silicones incl. volatile silicones | 80 |
| Oils, waxes and fats | 30 |
| Resins, film forming polymers | 30 |
| Colorants, colour additives | 25 |
| Emulsifying agents (e.g. cera alba) | 20 |
| Bulking agents | 15 |
| Thickeners | 12 |
| Ethanol (alcohol denat.) | 10 |
| Aqua | 10 |
| Humectants | 5 |
| Berry based ingredients | 0.01-80 |
| Perfume | 1 |
| Antioxidants | 1 |
| Preservatives, antimicrobials | 1 |

In the above composition, suitable isoparaffins include for example branched-chain isoparaffin (C11-C16), isododecane and isohexadecane. Typical silicones include for example cyclopentasiloxane and dimethicone. Film forming polymers include for example aluminium distearate and VP/hexadecane copolymer. Colour additives may include for example opacifying agents and pearlescent agents. Typical bulking agents include for example talc, kaolin, and zinc stearate. An example of a suitable thickener for the above composition is stearalkonium hectorite.

### Example 10. Skin oil (bi-phase or tri-phase)

| INGREDIENTS | Max. levels per phase% (w/w) |
|---|---|
| Ethanol (alcohol, alcohol denat.) | 90 |
| Water / aqua | 99 |
| Humectans (e.g. glycerin) | 20 |
| Plant extracts | 99.5 |
| Thickeners (e.g. dextrin palmitate) | 5 |
| Parfum | 5 |
| Additional ingredients | 5 |
| Preservatives, antimicrobials | 1.5 |
| Colorants | 1 |
| Oils (e.g. vegetable) | 99 |

In the above composition, "plant extracts" refer particularly to berry waters. However, berry extracts, fermented berry extracts (berry vinegar extracts), berry oils, and/or dry berry extracts or crushes may also be included. Berry waters may also replace water otherwise included in the composition. Additional ingredients may include for example antioxidants and colorants.

### Example 11. Hair / scalp care lotion

| INGREDIENTS | Max. levels % (w/w |
|---|---|
| Ethanol and/or isopropanol | 60 |
| Plant extracts | 95 |
| Polymers (e.g. PVP) | 10 |
| Hair conditioning agents | 5 |
| Oils, waxes and fats | 5 |
| Silicones (incl. volatile silicones) | 5 |
| Humectants (e.g. glycerin) | 5 |
| Additional ingredients | 5 |
| Emulsifying agents | 3 |
| Parfum | 3 |
| Preservatives, antimicrobials | 2 |
| Colorants | 1 |
| Aqua | to 100 |

In the above composition, "plant extracts" refer particularly to berry waters. Berry waters may also replace water otherwise included in the composition. However, berry extracts, fermented berry extracts (berry vinegar extracts), berry oils, and/or dry berry extracts or crushes may also be included. Hair conditioning agents include e.g. cationic polymers and cellulose derivatives. Oils may be vegetable and/or mineral oils. Waxes and fats include for example long chain alcohols. Silicones include e.g. cyclopentasiloxane, dimethicone, amodimethicone, and silanes (e.g. alkoxysilanes). Additional ingredients may include for example vitamins, chelating agents, UV filters and protein hydrolysates. Emulsifying agents include for example ethoxylated long chain alcohols.

### Example 12. Shampoo (liquid, cream, paste)

| INGREDIENTS | Max. levels % (w/w |
|---|---|
| Anionic surfactants | 30 |
| Plant extracts | 80 |
| Amphoteric surfactants | 20 |
| Non-ionic surfactants | 15 |
| Hair conditioning agents | 15 |
| Additional ingredients | 10 |
| Thickeners | 10 |
| Cationic surfactants | 5 |
| Parfum | 2 |
| Preservatives, antimicrobials | 1.5 |
| Chelating agents | 0.5 |
| Aqua | to 100 |

Anionic surfactants include in the above composition may include for example sodium/ammonium/TEA lauryl sulfates, and sodium/ammonium/TEA laureth sulfates. Plant extracts refer particularly to berry waters but berry extracts, fermented berry extracts or berry vinegar extracts, berry oils, and/or dry berry extracts or crushes may also be included. Berry waters may also replace water otherwise included in the composition. Amphoteric surfactants are for example betaine derivatives. As an example of non-ionic surfactants fatty alkanolamides may be mentioned. Hair conditioning agents include for example silicones including volatile silicones (e.g. cyclopentasiloxane, dimethicone, amodimethicone) and silanes (e.g. alkoxysilanes), cysteine derivatives, cellulose derivatives, and fatty acid esters. Additional ingredients in the above composition may include for example UV filters, pearlescent agents and opacifying agents. Thickeners are for example propylene glycol and PEG, while cationic surfactants comprise e.g. stearamidopropyl dimethylamine and behentrimonium chloride.

### Example 13. Shampoo plus conditioner

| INGREDIENTS | Max. levels % (w/w) |
|---|---|
| Plant extracts | 90 |
| Anionic surfactants | 20 |
| Non-ionic surfactants | 20 |
| Amphoteric surfactants | 20 |
| Thickeners | 10 |
| Oils and waxes | 10 |
| Silicones incl. volatile silicones | 10 |
| Cationic surfactants | 5 |
| Cationic polymers | 5 |
| Humectants, emollients | 5 |
| Additional ingredients | 5 |
| Parfum | 2 |
| Preservatives, antimicrobials | 1.5 |
| Colorants | 1 |
| Chelating agents | 0.5 |
| Aqua | to 100 |

Anionic surfactants in the above composition may include for example sodium laureth sulfate, non-ionic surfactants for example alkyl polyglucosides and amphoteric surfactants for example betaine derivatives. Cationic surfactants may include e.g. behentrimonium chloride. Plant extracts refer particularly to berry waters but berry extracts, fermented berry extracts or berry vinegar extracts, berry oils, and/or dry berry extracts or crushes or mixtures thereof may also be included. Berry waters may also replace water otherwise included in the composition. Thickeners include e.g. long chain alcohols and stearyl alcohol. Oils include both vegetable and/or mineral oils. Silicones may include volatile silicones (e.g. cyclopentasiloxane, dimethicone, amodimethicone) and silanes (e.g. alkoxysilanes). One example of cationic polymers is polyquaternium-11. Additional ingredients in the above composition may include for example UV filters and pearlescent agents.

### Example 14. Hair conditioner

| INGREDIENTS | Max. levels % (w/w) |
|---|---|
| Plant extracts | 80 |
| Silicones incl. volatile silicones | 20 |
| Ethanol and/or isopropanol | 15 |
| Amphoteric surfactants | 15 |
| Emulsifying agents | 10 |
| Emollients, humectants | 10 |
| Thickeners | 10 |
| Additional ingredients | 10 |
| Cationic surfactants | 5 |
| Polymers, resins | 5 |
| Parfum | 3 |
| UV filters | 2 |
| Preservatives, antimicrobials | 1.5 |
| Colorants | 1 |
| Aqua | to 100 |

In the above composition plant extracts refer particularly to berry waters but berry extracts, fermented berry extracts (berry vinegar extracts), berry oils, and/or dry berry extracts or crushes or mixtures thereof may also be included. Berry waters may also replace water otherwise included in the composition. Silicones may include volatile silicones (e.g. cyclopentasiloxane, dimethicone, amodimethicone) and silanes (e.g. alkoxysilanes). Emulsifying agents may include.g. ceteth-30 and cetyl alcohol. Emollients and humectants typically include e.g. glycerine and propylene glycol. Examples of thickeners include carbomer and hydroxyethylcellulose. Additional ingredients may include e.g. proteins, chelating agents and pearlescent agents. An example of cationic surfactants is behentrimonium chloride. Polymers and resins may include e.g. polyquaternium-10, polyquaternium-11, and butyl ester of PVM/MA copolymer.

As will be understood from the preceding description of the present invention and the illustrative experimental examples, the present invention can be described by reference to the following embodiments:
1. A cosmetic composition comprising cosmetically acceptable substances and at least one cosmetically acceptable berry fraction, wherein the at least one cosmetically acceptable berry fraction comprises a berry industry side stream or an extract from a berry industry side stream from processing of Nordic berries, wherein the berry industry side stream or the extract from a berry industry side stream is selected from one or more of the following:
   - a residue from concentration of berry juice;
   - a water soluble extract from press residue of berry juice production;
   - an oil soluble extract from press residue of berry juice production;
   - fermented extracts of berry leaves;
   - fermented extracts from press residue of berry juice production; and
   - peel or seed waste in dried and crushed form.
2. The composition according to embodiment 1, wherein the berry industry side stream is a residue from concentration of berry juice, preferably an evaporation residue from concentration of berry juice or an evaporation residue from spray drying of berry juice.
3. The composition according to embodiment 1 or 2, wherein the Nordic berries are selected from the group consisting of lingonberry (*Vaccinium vitis-idaea*), bilberry (*Vaccinium myrtillus*), cloudberry (*Rubus chamaemorus*), cranberry (*Vaccinium oxycoccus* / *Oxycoccus palustris*), small cranberry (*Vaccinium microcarpum*), bog bilberry (*Vaccinium uliginosum*)*,* raspberry (*Rubus idaeus*), crowberry (*Empetrum nigrum*), rowanberry (*Sorbus aucuparia, Sorbus fennica*), sea buckthorn (*Hippophaë rhamnoides*), blackcurrant (*Ribes nigrum*), redcurrant (*Ribes rubrum*), white currant (*Ribes rubrum* f. *leucocarpum*), greencurrant (*Ribes nigrum*), gooseberry (*Ribes uva-crispa*), wild recurrant (*Ribes spicatum*), mountain currant (*Ribes alpinum*)*,* aronia berry (*Aronia mitschurinii*), saskatoon (*Amelanchier alnifolia*)*,* Arctic bramble (*Rubus arcticus),* stone bramble (*Rubus saxatilis*), strawberry (*Fragaria x ananassa*)*,* blackberry (*Rubus fruticosus*)*,* bearberry (*Arctostaphylos uva-ursi*), alpine bearberry (*Arctostaphylos alpine*), wild strawberry (*Fragaria vesca*,) juniper berry (*Juniperus communis*), wild rose (*Rosa acicularis*)*,* dog rose (*Rosa canina*)*,* cinnamon rose (*Rosa cinnamomea* / *Rosa majalis*), glaucous dog rose (*Rosa dumalis*), blackthorn (*Prunus spinosa*), Cornelian cherry (*Cornus mas*), bunchberry (*Cornus suecica*)*,* dewberry (*Rubus caesius*), Finnish whitebeam (*Hedlundia hybrida*)*,* and bird cherry (*Prunus padus*)*.*
4. The composition according to any one of the preceding embodiments, wherein the berries are selected from lingonberry (*Vaccinium vitis-idaea*), bilberry (*Vaccinium myrtillus*), cloudberry (*Rubus chamaemorus*), cranberry (*Vaccinium oxycoccus* / *Oxycoccus palustris),* small cranberry *(Vaccinium microcarpum),* crowberry (*Empetrum nigrum*), and sea buckthorn (*Hippophaë rhamnoides*)*.*
5. The composition according to any one of the preceding embodiments, wherein the cosmetic composition is a skin care cream, skin care lotion, skin care gel, skin oil, cosmetic tonic water, cleansing gel, a foundation cream, a lip care stick, a lip care cream, a lip care gel, a lip colour stick, cream or gel, zinc oxide based cream, a roll-on antiperspirant, a foundation cream, a mascara, shampoo, conditioner, hair mask, hair spray, hair wax, hair gel, hair mousse, hair pomace, hair coloring composition (permanent, semi-permanent, or direct dye), permanent lotion, oxiding agent for coloring of hair, or heat protection composition for hair.
6. The cosmetic composition according to any one of the preceding embodiments, wherein the amount of cosmetically acceptable berry fraction(s) is at most 99.5% (w/w) of the total weight of the cosmetic composition.
7. The cosmetic composition according to any one of the preceding embodiments, which contains an amount of cosmetically acceptable berry fraction(s), which consist or consist essentially of berry industry side streams or extracts therefrom, wherein the amount is at least 25%, preferably at least 50%, of the total weight of the cosmetic composition.
8. The cosmetic composition according to any one of the preceding embodiments, which is a skin care cream, oil, lotion or gel, a cosmetic tonic water, a cleansing gel, a hair care product, a make-up product, or an antiperspirant roll-on, wherein the amount of cosmetically acceptable berry fraction(s) is 0.01-99.5% (w/w), preferably 0.01-95%, 0.01-70%, 0.01-60%, 0.01-50%, 0.01-40%, 0.01-30%, 0.01-20%, 0.01-10% or 0.01-5%, of the total weight of the composition.
9. The cosmetic composition according to any one of embodiments 1 to 8, which is a zinc oxide based cream, wherein the amount of cosmetically acceptable berry fraction(s) is 0.01-60% (w/w), preferably 0.01-50%, 0.01-40%, 0.01-30%, 0.01-20%, 0.01-10%, more preferably 0.01-5% or 0.1-5%, of the total weight of the composition.
10. The cosmetic composition according to any one of embodiments 1 to 8, which is a skin oil, wherein the amount of cosmetically acceptable berry fraction(s) is 0.01-100% or 0.01-99.5% (w/w), preferably 0.01-80%, 0.01-70%, 0.01-60%, 0.01-50%, 0.01-40%, more preferably 0.01-30% or 0.5-20%, of the total weight of the composition.
11. The cosmetic composition according to any one of embodiments 1 to 8, which is a lip care or lip colour product, such as a stick, cream or gel, wherein the amount of cosmetically acceptable berry fraction(s) is 0.01-20% (w/w), preferably 0.01-15%, 0.01-10%, or 0.01-5%, more preferably 0.01-3% or 0.01-2%, of the total weight of the composition.
12. The cosmetic composition according to any one of embodiments 1 to 8, which is a foundation, wherein the amount of cosmetically acceptable berry fraction(s) is 0.01-90% (w/w), preferably 0.01-80%, 0.01-70%, 0.01-60%, 0.01-50%, 0.01-40%, 0.01-30%, more preferably 0.01-20%, 0.01-10% or 0.01-5%, of the total weight of the composition.
13. The cosmetic composition according to any one of embodiments 1 to 8, which is a mascara, wherein the amount of cosmetically acceptable berry fraction(s) is 0.01-80% (w/w), preferably 0.01-50%, 0.01-40%, 0.01-30%, 0.01-20%, 0.01-10%, more preferably 0.01-5% or 0.1-5%, of the total weight of the composition.
14. The cosmetic composition according to any one of embodiments 1 to 8, which is a hair care product, such as shampoo, conditioner, hair mask, hair spray, hair wax, hair gel, hair mousse, hair pomace, hair coloring composition (permanent, semi-permanent, or direct dye), permanent lotion, oxiding agent for coloring of hair, heat protection composition for hair, or a beard and moustache cleansing, conditioning or styling product, wherein the amount of cosmetically acceptable berry fraction(s) is 0.01-80% (w/w), preferably 0.01-50%, 0.01-40%, 0.01-30%, 0.01-20%, 0.01-10%, 0.01-5% or 0.1-5%, of the total weight of the composition.
15. A method for utilizing side streams of berry industry wherein said side streams are selected from peel and seed waste, berry leaves, press residues from pressing of berries, and residues from concentration of berry juice, and wherein said side streams are included in cosmetic compositions as such or after modification steps selected from concentration, different types of extraction processes, pressing, fermentation, filtration and adding preservatives.
16. The method according to embodiment 15, wherein the side stream is a residue from concentration of berry juice, preferably an evaporation residue from a concentration process of raw berry juice to concentrated berry juice or an evaporation residue from spray drying of concentrated berry juice.

While the forgoing examples are illustrative of the principles of the present invention in one or more particular applications, it will be apparent to those of ordinary skill in the art that numerous modifications in form, usage and details of implementation can be made without the exercise of inventive faculty, and without departing from the principles and concepts of the invention. Accordingly, it is not intended that the invention be limited, except as by the claims set forth below.

The verbs "to comprise" and "to include" are used in this document as open limitations that neither exclude nor require the existence of also un-recited features. The features recited in depending claims are mutually freely combinable unless otherwise explicitly stated. Furthermore, it is to be understood that the use of "a" or "an", that is, a singular form, throughout this document does not exclude a plurality.

### INDUSTRIAL APPLICABILITY

At least some embodiments of the present invention find industrial application in cosmetic industry, providing the use of raw materials, which are side streams or by-products from berry industry.

### CITATION LIST

### Patent Literature

WO 2018/055244 A1
US 5,853,730

## Claims

1. A cosmetic composition comprising cosmetically acceptable substances and at least one cosmetically acceptable berry fraction, wherein the at least one cosmetically acceptable berry fraction comprises a berry industry side stream or an extract from a berry industry side stream from processing of Nordic berries, **characterized in that** the berry industry side stream comprises or is an evaporation residue from concentration of berry juice or an evaporation residue from spray drying of berry juice, in particular condensated vaporized water from concentration of berry juice and/or condensated vaporized water from spray drying of berry juice.

2. The composition according to claim 1, wherein the Nordic berries are selected from the group consisting of lingonberry (*Vaccinium vitis-idaea*)*,* bilberry (*Vaccinium myrtillus*), cloudberry (*Rubus chamaemorus*)*,* cranberry (*Vaccinium oxycoccus* / *Oxycoccus palustris*)*,* small cranberry (*Vaccinium microcarpum*), bog bilberry (*Vaccinium uliginosum*)*,* raspberry (*Rubus idaeus*)*,* crowberry (*Empetrum nigrum*), rowanberry (*Sorbus aucuparia, Sorbus fennica*), sea buckthorn (*Hippophaë rhamnoides*), blackcurrant (*Ribes nigrum*)*,* redcurrant (*Ribes rubrum*), white currant (*Ribes rubrum* f. *leucocarpum*), greencurrant (*Ribes nigrum*), gooseberry (*Ribes uva-crispa*), wild recurrant (*Ribes spicatum*)*,* mountain currant (*Ribes alpinum*)*,* aronia berry (*Aronia mitschurinii*)*,* saskatoon (*Amelanchier alnifolia*)*,* Arctic bramble (*Rubus arcticus*)*,* stone bramble (*Rubus saxatilis*)*,* strawberry (*Fragaria x ananassa*)*,* blackberry (*Rubus fruticosus*)*,* bearberry (*Arctostaphylos uva-ursi*), alpine bearberry (*Arctostaphylos alpine*), wild strawberry (*Fragaria vesca*,) juniper berry (*Juniperus communis*), wild rose (*Rosa acicularis*)*,* dog rose (*Rosa canina*)*,* cinnamon rose (*Rosa cinnamomea* / *Rosa majalis*), glaucous dog rose (*Rosa dumalis*)*,* blackthorn (*Prunus spinosa*), Cornelian cherry (*Cornus mas*), bunchberry (*Cornus suecica*)*,* dewberry *(Rubus caesius*), Finnish whitebeam (*Hedlundia hybrida*), and bird cherry (*Prunus padus*)*.*

3. The composition according to claim 1 or 2, wherein the berries are selected from lingonberry (*Vaccinium vitis-idaea*), bilberry (*Vaccinium myrtillus*)*,* cloudberry (*Rubus chamaemorus*)*,* cranberry (*Vaccinium oxycoccus* / *Oxycoccus palustris*), small cranberry (*Vaccinium microcarpum*), crowberry (*Empetrum nigrum*), and sea buckthorn (*Hippophaë rhamnoides*)*.*

4. The composition according to any one of the preceding claims, wherein the cosmetic composition is a skin care cream, skin care lotion, skin care gel, skin oil, cosmetic tonic water, cleansing gel, a foundation cream, a lip care stick, a lip care cream, a lip care gel, a lip colour stick, cream or gel, zinc oxide based cream, a roll-on antiperspirant, a foundation cream, a mascara, shampoo, conditioner, hair mask, hair spray, hair wax, hair gel, hair mousse, hair pomace, hair coloring composition (permanent, semi-permanent, or direct dye), permanent lotion, oxidizing agent for coloring of hair, or heat protection composition for hair, preferably a skin care cream, skin care lotion, skin care gel, skin oil, cosmetic tonic water, cleansing gel, foundation cream, zinc oxide based cream, roll-on antiperspirant or mascara.

5. The cosmetic composition according to any one of the preceding claims, wherein the amount of cosmetically acceptable berry fraction(s) is at most 99.5% (w/w) of the total weight of the cosmetic composition.

6. The cosmetic composition according to any one of the preceding claims, which contains an amount of cosmetically acceptable berry fraction(s), which consist or consist essentially of berry industry side streams or extracts therefrom, wherein the amount is at least 25%, preferably at least 50%, of the total weight of the cosmetic composition.

7. The cosmetic composition according to any one of the preceding claims, which is a skin care cream, oil, lotion or gel, a cosmetic tonic water, a cleansing gel, a hair care product, a make-up product, or an antiperspirant roll-on, wherein the amount of cosmetically acceptable berry fraction(s) is 0.01-99.5% (w/w), preferably 0.01-95%, 0.01-70%, 0.01-60%, 0.01-50%, 0.01-40%, 0.01-30%, 0.01-20%, 0.01-10% or 0.01-5%, of the total weight of the composition.

8. The cosmetic composition according to any one of claims 1 to 7, which is a zinc oxide based cream, wherein the amount of cosmetically acceptable berry fraction(s) is 0.01-60% (w/w), preferably 0.01-50%, 0.01-40%, 0.01-30%, 0.01-20%, 0.01-10%, more preferably 0.01-5% or 0.1-5%, of the total weight of the composition.

9. The cosmetic composition according to any one of claims 1 to 7, which is a skin oil, wherein the amount of cosmetically acceptable berry fraction(s) is 0.01-100% or 0.01-99.5% (w/w), preferably 0.01-80%, 0.01-70%, 0.01-60%, 0.01-50%, 0.01-40%, more preferably 0.01-30% or 0.5-20%, of the total weight of the composition.

10. The cosmetic composition according to any one of claims 1 to 7, which is a lip care or lip colour product, such as a stick, cream or gel, wherein the amount of cosmetically acceptable berry fraction(s) is 0.01-20% (w/w), preferably 0.01-15%, 0.01-10%, or 0.01-5%, more preferably 0.01-3% or 0.01-2%, of the total weight of the composition.

11. The cosmetic composition according to any one of claims 1 to 7, which is a foundation, wherein the amount of cosmetically acceptable berry fraction(s) is 0.01-90% (w/w), preferably 0.01-80%, 0.01-70%, 0.01-60%, 0.01-50%, 0.01-40%, 0.01-30%, more preferably 0.01-20%, 0.01-10% or 0.01-5%, of the total weight of the composition.

12. The cosmetic composition according to any one of claims 1 to 7, which is a mascara, wherein the amount of cosmetically acceptable berry fraction(s) is 0.01-80% (w/w), preferably 0.01-50%, 0.01-40%, 0.01-30%, 0.01-20%, 0.01-10%, more preferably 0.01-5% or 0.1-5%, of the total weight of the composition.

13. The cosmetic composition according to any one of claims 1 to 7, which is a hair care product, such as shampoo, conditioner, hair mask, hair spray, hair wax, hair gel, hair mousse, hair pomace, hair coloring composition (permanent, semi-permanent, or direct dye), permanent lotion, oxidizing agent for coloring of hair, heat protection composition for hair, or a beard and moustache cleansing, conditioning or styling product, wherein the amount of cosmetically acceptable berry fraction(s) is 0.01-80% (w/w), preferably 0.01-50%, 0.01-40%, 0.01-30%, 0.01-20%, 0.01-10%, 0.01-5% or 0.1-5%, of the total weight of the composition.

14. A method for utilizing side streams of berry industry wherein said side streams are residues from concentration of berry juice, selected from condensated vaporized water from a concentration process of raw berry juice to concentrated berry juice, condensated vaporized water from spray drying of concentrated berry juice, and their combinations, and wherein said side streams are included in cosmetic compositions as such or after modification steps selected from concentration, different types of extraction processes, fermentation, filtration and adding preservatives.
